# EUROPEAN PATENT SPECIFICATION

(11) **EP 3 865 060 B1**
(45) Date of publication and mention of the grant of the patent: **30.11.2022**
(21) Application number: 20157079.3
(22) Date of filing: 13.02.2020
(51) Int. Cl.: A61B 5/026, A61B 5/0295

(54) **COMPUTER-IMPLEMENTED METHOD FOR GENERATING AN ANNOTATED PHOTOPLETHYSMOGRAPHY (PPG) SIGNAL**
COMPUTERIMPLEMENTIERTES VERFAHREN ZUR ERZEUGUNG EINES ANNOTIERTEN PHOTOPLETHYSMOGRAPHIE(PPG)-SIGNALS
PROCÉDÉ MIS EN UVRE PAR ORDINATEUR POUR GÉNÉRER UN SIGNAL DE PHOTOPLÉTHYSMOGRAPHIE ANNOTÉ (PPG)

(43) Date of publication of application: 18.08.2021
(73) Proprietor: Qompium, 3500 Hasselt (BE)
(72) Inventor: Vanvinckenroye, Amaury Marie-Christine Christophe, 4260 Braives (BE); De Witte, Glenn, 3020 Herent (BE); Lambein, Xavier Marie Christian Fernand, 1330 Rixensart (BE); Leysen, Kobe Roger Marijke, 3740 Bilzen (BE); Grieten, Lars, 3690 Zutendaal (BE); Van der Auwera, Jo, 2450 Meerhout (BE); Van Gorp, Bie Josephus Constantia, 2460 Tielen (BE)
(74) Representative: IP HILLS NV

(56) References cited:
- WO-A1-2018/002541
- US-A1- 2017 347 899
- US-A1- 2019 117 096
- US-A1- 2019 307 337
- FRANCESCO RUNDO ET AL: "An Advanced Bio-Inspired PhotoPlethysmoGraphy (PPG) and ECG Pattern Recognition System for Medical Assessment", SENSORS, vol. 18, no. 2, 30 January 2018 (2018-01-30), page 405, XP055558039, DOI: 10.3390/s18020405

## Description

### Field of the Invention

The present invention generally relates to the generation of annotated signals indicative for physiological parameters. More particularly, the invention relates to the production of photoplethysmography signals, abbreviated PPG signals, that are reliably annotated with annotations indicative for instance for the quality of the PPG signal measurement, the quality of the annotation, and/or clinical annotations like heart rhythm related annotations (regular sine rhythm and various irregular rhythm annotations) or even non-rhythm clinical annotations.

### Background of the Invention

Photoplethysmography (PPG) is an optical technique that allows to monitor one or more physiological parameters by detecting blood-volume changes in peripheral circulation. PPG makes use of light absorption by blood to track these volumetric changes. When a light source illuminates the skin, the reflected light varies as blood flows. A light sensor then converts these variations in light reflection into a digital signal, the so-called PPG signal. PPG signals are typically recorded using a pulse oximeter or photodetector, for instance the camera integrated in an electronic device like a person's smartphone, smartwatch or other smart wearable or non-wearable device.

A distinction is made between remote PPG and contact PPG. Remote PPG is non-obtrusive for the monitored person but poses major challenges to signal detection and signal processing. As a consequence, the use of remote PPG remains limited to everyday applications like leisure or fitness as its accuracy and reliability are insufficient for medical applications. Contact PPG, wherein the measurement components are in direct contact with the skin, results in a more reliable, more accurate PPG signal that facilitates medical diagnosis.

In a medical context, contact PPG is mainly used for atrial fibrillation (AF) risk detection, the most common cardiac rhythm disorder. However, contact PPG is not yet recognized as a reliable technique to observe other cardiac rhythm disorders. Today, cardiac rhythm disorders are still typically detected via an electrocardiogram (ECG) obtained for instance by a Holter monitoring system.

Signals obtained by PPG sensors are rather sensitive to the recording conditions. Sub-optimal conditions can lead to lower or insufficient signal quality. Artefacts in PPG signals may result from motion, bad positioning of the skin with respect to the light sensor, ambient light interference, PPG waveform inversion, etc. In order to classify PPG signal portions as regular, irregular (cardiac rhythm disorder), or of insufficient quality, machine learning technology may be deployed. To become a performant classifier, the machine learning technology however must be trained with training data, i.e. PPG signals that are reliably annotated.

Existing public databases contain annotated electrocardiogram (ECG) signals. Annotated ECG signals however do not allow to train PPG signal classifiers.

PPG technology is not recognized as the gold standard for heart rhythm identification and as such, not a lot of annotated PPG data is available. Moreover, because of the artefacts often present in PPG data, it is a complex task to annotate PPG signals, which can result in inconsistent annotations. For instance, some people may be more confident in annotating lower quality signal data than others, leading to subjective differences in annotations.

On PPG signals only atrial fibrillation (AF) can reliably be identified. PPG signals can hardly be used to annotate other heart rhythms. ECG seems to be the only alternative to obtain reliable expert annotations. Furthermore, ECG signals are less subject to noise and medical technicians are usually more confident with observing ECG traces compared to observing PPG traces. This has inspired people to map or copy expert-based ECG annotations onto simultaneously obtained PPG signals.

United States Patent Application US 2019/0328243 A1 entitled "Methods and Systems For Determining Abnormal Cardiac Activity" describes detection of abnormal cardiac activity by recording ECG and PPG signals, slicing these signals, and classifying the signal slices using neural networks. US 2019/0328243 A1 however remains vague on how the neural networks must be trained. According to US 2019/0328243 A1, the neural networks are trained through "known subject information". Such known subject information does not comprise PPG signals annotated through ECG-PPG annotation mapping. Some paragraphs in US 2019/0328243 A1 suggest that the neural networks are also able to classify signal slices as "noisy" or "bad quality" slices but the detection of bad quality portions is not a result of ECG-PPG annotation mapping.

The article "Wrist-Located Optical Device for Atrial Fibrillation Screening: A Clinical Study on Twenty Patients" from authors M. Lemay et al. describes beat-to-beat alignment (synchronization) of an ECG signal and PPG signal, semi-synchronously collected using different devices on a single person. Once the time base of the PPG signal is transformed to match the time base of the ECG signal, expert annotations of rhythm epochs in the ECG signal are automatically projected onto the PPG signal. This leads to automated annotation of each interbeat interval in the PPG signal.

M. Lemay et al. however do not detect/discard bad quality portions of the PPG signal before projecting the ECG annotations on the PPG signal. This results in inaccurately annotated PPG signals that cannot be used as reliable training data to train a neural network that will later be used for PPG signal classification and medical diagnosis.

In general, the approach available in literature consists in copying annotations from ECG to PPG irrespective of how much noise is present or how many artefacts are present in the PPG signal. When such annotated PPG signals are used to train a neural network, this leads to a neural network that always has as outcome a medical diagnosis, even when there is a lot of noise present in the input signal. This may lead to mistakes and consequently unreliable medical diagnosis.

In addition, it is unclear from the cited article of M. Lemay et al. how individual ECG beats are mapped onto individual PPG beats.

United States Patent Application US 2019/0307337 A1 entitled "Methods and Apparatus for Self-Calibrating Non-Invasive Cuffless Blood Pressure Measurements" describes a cuffless method for blood pressure measurement that requires congruent measurement of ECG and PPG signals Through a cross-correlation function, sub-millisecond accuracy is achieved in calibrating time differences between the ECG and PPG signals. It looks like US 2019/0307337 A1 achieves to map ECG beats with PPG beats over a long period of time through regular recalibration. US 2019/0307337 A1 however does not map ECG annotations onto PPG signal portions, does not envisage to generate reliable training data, and does not suggest insufficient quality portion detection in the PPG signal.

International patent application WO 2018/002541 A1 entitled "Device For Detecting At Least One Cardiac Rhythm Disturbance" describes parallel measurement of ECG and PPG signals, and peak detection in small time windows. RR intervals are calculated, and a variable calculated from the RR intervals is used to classify abnormal heartrates. WO 2018/002541 A1 does not rely on a neural network to classify abnormal cardiac activity and consequently also does not solve the problem of generating reliably annotated PPG signals to train such neural network.

### Summary of the Invention

It is an object of the present invention to disclose a method and system to annotate a PPG signal that overcomes or mitigates one or more of the above-identified drawbacks of existing solutions. More precisely, it is an objective to disclose a method for annotating a PPG signal allowing to generate an annotated PPG signal that is more reliable and more consistent such that the annotated PPG signal can be used in applications like training a neural network or comparing PPG pre-processing techniques. The invention shall ultimately contribute to more reliable medical diagnosis, detection of a wider range of heart rhythm disorders or other clinical disorders as PPG classifiers trained using annotated PPG signals produced according to the invention will perform better.

According to a first aspect of the invention, the above identified object is achieved through the computer-implemented method for generating an annotated photoplethysmography signal, abbreviated annotated PPG signal, defined by claim 1, the method comprising:
- recording an electrocardiogram signal, abbreviated ECG signal, using an ECG monitoring system;
- recording a photoplethysmography signal, abbreviated PPG signal, semi-synchronously with the recording of the ECG signal, using a contact PPG sensor;
- annotating segments in the ECG signal either algorithm-based or expert-based, resulting in ECG segment annotations;
- time-aligning the PPG signal and the ECG signal;
- detecting cardiac beats in the ECG signal, named ECG beats;
- detecting cardiac beats in the PPG signal, named PPG beats;
- pairing the ECG beats with the PPG beats;
- deriving annotations for PPG signal segments based on the nature of the ECG segment annotations and on how the ECG beats can be paired with the PPG beats; and
- annotating the PPG signal segments using the annotations, thereby generating the annotated PPG signal.

Thus, embodiments of the invention concern a more advanced method for ECG-PPG annotation mapping, resulting in a more reliable, consistent annotated PPG signal. The ECG signal and PPG signal are semi-synchronously recorded using different devices on a single person or animal. The ECG signal is obtained by an ECG monitoring system, for example a Holter monitoring system. The PPG signal is obtained by a PPG sensor, for example a pulse oximeter or smartphone camera, in direct contact with the person or animal skin. Semi-synchronously in the context of the present invention means that the ECG monitoring system is connected to the person or animal and the ECG measurement is launched seconds or minutes before the PPG sensor is making contact with the person or animal skin and the PPG measurement is started, or vice versa. A substantial part of the ECG signal and PPG signal however overlap in time and this time-overlapping part of the ECG and PPG signals is the relevant portion of the signals in view of the invention.

The ECG signal is annotated either algorithm-based or expert-based. This means that annotations are assigned to segments of the ECG signal either automatically based on an ECG annotation algorithm or manually based on the expertise of one or plural people like cardiologists, medical technicians, data scientists, nurses, etc. The segments can be fixed length segments like for instance segments of 1 second, or variable length segments like for instance inter beat intervals.

The ECG and PPG signals, semi-synchronously obtained on a single person or animal, are then aligned. This implies that the ECG and PPG signals are synchronised in time with milliseconds accuracy. The alignment compensates for the offset that exists in between the recordings of the ECG and PPG signals, and it compensates for the drift in clocks between the ECG monitoring system and PPG sensor device, if any.

Cardiac beats are then detected in the ECG signal and PPG signal, either algorithm-based or expert-based, and a pairing PPG beat is searched for every ECG beat. The pairing of PPG beats with ECG beats is typically based on the timing of the beats in the time-aligned ECG and PPG signals. In a straightforward implementation, the pairing shall result in each ECG beat being associated with the closest PPG beat, i.e. the PPG beat that is closest in time to the ECG beat after alignment of the ECG and PPG signals.

Embodiments of the invention take advantage of this pairing between an ECG beat and PPG beat to determine the annotation for a corresponding PPG signal segment. However, the ECG annotation is not merely copied onto the pairing PPG beat or onto a PPG segment comprising the pairing PPG beat. Both the nature of the ECG segment annotation and how the ECG beat pairs with the PPG beat will be considered in the determination of the annotation of the corresponding PPG segment. If no PPG beat can be paired with an ECG beat, the ECG segment annotation around that ECG beat may not be copied onto the corresponding PPG segment. If no ECG beat can be paired with a PPG beat, the ECG segment annotation around that PPG beat (i.e. the ECG segment annotation of an ECG segment time-corresponding with a portion of the PPG signal comprising the PPG beat) may not be copied onto the corresponding PPG segment. Such PPG segments may be attributed annotations that differ from the corresponding ECG segment annotation, like for instance the annotation "insufficient quality", "undefined label", or a different, new annotation. The attributed PPG segment annotation hence will depend on the pairing of ECG beats with PPG beats, for instance the successful pairing or unsuccessful or a quality value assigned to the pairing if the success of pairing is qualitatively assessed by an algorithm, as well as on the nature of the corresponding ECG segment annotation. Nature of the ECG segment annotation refers to the type of annotation, e.g. rhythm based annotation, non-rhythm clinical annotation, quality based annotation or other type of annotation, or may alternatively refer to the actual annotation itself, e.g. "SR" (regular sine rhythm), "AF" (atrial fibrillation), "PAC" (premature atrial contraction), "SA" (sleep apnea), "IQ" (insufficient quality), "UD" (undefined), etc. By not preserving the ECG annotation and replacing the ECG annotation with an annotation that is determined based on how the ECG beat pairs with the PPG beat, the PPG signal annotation becomes more reliable and more consistent.

A machine learning tool that is trained with PPG signals generated according to an embodiment of the present invention is able to more reliably detect various heart rhythms or other clinical disorders and to identify insufficient quality portions in a PPG signal. Consequently, a machine learning tool trained using annotated PPG signals generated according to an embodiment of the present invention can inform that another measurement is needed in order to make a reliable medical diagnosis, which will not happen in case PPG signals are annotated by merely copying time-aligned ECG annotations.

In embodiments of the computer-implemented method for generating an annotated PPG signal according to the invention, as defined by claim 2, deriving annotations for PPG signal segments comprises:
- copying an annotation of an ECG segment onto a time-corresponding PPG segment when an ECG beat can be paired with a PPG beat for said segment.

In such embodiments, successful pairing of an ECG beat with a PPG beat, i.e. the ability to match a PPG beat with an ECG beat based on the timing of these beats in the time-aligned ECG and PPG signals, shall result in the annotation of the ECG segment where the ECG beat is lying in being copied onto the time-corresponding PPG segment.

In embodiments of the computer-implemented method for generating an annotated PPG signal according to the invention, defined by claim 3, deriving annotations for PPG signal segments comprises:
- marking a PPG segment as insufficient quality segment when no ECG beat can be paired with a PPG beat for the segment or when no PPG beat can be paired with an ECG beat for the segment.

In such embodiments, the annotation of an ECG segment shall not be copied onto the time-corresponding PPG segment when the ECG beat detected for that ECG segment cannot be paired with a PPG beat (false negative), for instance because the beat detection algorithm fails to detect a beat in the time-corresponding PPG segment or when a human expert does not identify a beat in the time-corresponding PPG segment. Alternatively or supplementary, in such embodiments, the annotation of an ECG segment shall not be copied onto the time-corresponding PPG segment when a PPG beat cannot be paired with an ECG beat (false positive), for instance because the beat detection algorithm incorrectly detects a beat in the time-corresponding PPG segment or when a human expert incorrectly identifies a beat in the time-corresponding PPG segment. Instead of copying the ECG annotation, the PPG segment will be annotated as "insufficient quality segment".

In embodiments of the computer-implemented method for generating an annotated PPG signal according to the invention, defined by claim 4, deriving annotations for PPG signal segments comprises:
- copying an annotation of an ECG segment onto a time-corresponding PPG segment when an ECG beat cannot be paired with a PPG beat for the segment or attributing a new annotation to a time-corresponding PPG segment when an ECG beat cannot be paired with a PPG beat for the segment.

Indeed, in certain circumstances, an ECG beat may not be visible in the PPG signal, for instance when a premature cardiac contraction occurs and leads to low blood ejection from the heart. Rather than annotating the time-corresponding PPG segment as "insufficient quality", the annotation of the ECG segment may be preserved, or a new annotation may be attributed to the time-corresponding PPG segment. For example, during an episode of atrial fibrillation, some cardiac contractions will result in reduced cardiac output, and a beat will be visible in the ECG signal but not in the PPG signal. However, the ECG annotation "atrial fibrillation" is maintained for the time-corresponding PPG segment. Similarly, when a normal sine rhythm is interrupted by a premature cardiac contraction, there might also be a reduced cardiac output resulting in a visible premature beat in the ECG signal but being invisible in the PPG signal. The time-corresponding PPG segment may be attributed the new "invisible premature contraction" annotation in such case.

In embodiments of the computer-implemented method for generating an annotated PPG signal according to the invention, as defined by claim 5, deriving annotations for PPG signal segments comprises:
- marking a PPG segment as undefined segment when no ECG beat can be paired with a PPG beat for the segment because a time-corresponding ECG segment has insufficient quality.

Indeed, the inability to pair an ECG beat with a PPG beat may also result from low quality of the ECG measurement for the considered segment. In such case, the ECG annotation cannot be reliably copied onto the time-corresponding PPG segment, and embodiments of the invention may be configured to annotate the time-corresponding PPG segment as "undefined". The poor quality of the ECG measurement does not necessarily imply that also the time-corresponding PPG measurement is of bad quality, but copying the expert-based or algorithm-based ECG annotation onto the time-corresponding PPG segment would result in unreliable, inaccurate PPG annotation.

In embodiments of the computer-implemented method for generating an annotated PPG signal according to the invention, as defined by claim 6, pairing the ECG beats with the PPG beats comprises:
- selecting an ECG beat;
- determining an ECG signal portion comprising the ECG beat and no other ECG beats;
- determining a PPG signal portion time-aligned with the ECG signal portion;
- identifying a PPG beat lying within the PPG signal portion;
- associating the PPG beat with the ECG beat.

Thus, individual ECG beats may also be mapped onto the PPG signal, for instance when no expert annotation is available or when a PPG beat detection algorithm gives inaccurate results. One way to realize the mapping may comprise isolating an ECG beat in a time window or ECG signal portion that comprises no other ECG beats, considering the time-corresponding portion in the time-aligned PPG signal and detecting a PPG beat in that time-corresponding PPG signal portion. The ECG beat is then mapped onto the detected PPG beat or - in other words - the detected PPG beat is paired with the ECG beat.

In embodiments of the computer-implemented method for generating an annotated PPG signal according to the invention, as defined by claim 7, detecting the PPG beats comprises detecting local maxima in the PPG signal.

Thus, in an example embodiment of the invention, local maxima are detected in the PPG signal in order to detect cardiac beats. In such embodiment, for every ECG beat a corresponding PPG beat is searched for in the aligned PPG signal by looking for a local maximum in the PPG signal. As there might subsist some small misalignment between an ECG beat and the corresponding PPG beat, the local maximum is preferably searched for in a small window, for instance having a total length of 100 milliseconds, i.e. 50 milliseconds left of (before) and 50 milliseconds right of (after) the ECG beat. The skilled person will appreciate that plural local maxima may be detected within such window. Not every local maximum represents a PPG beat, and additional logic will be required to select the local maximum best representing the PPG beat, for example the highest local maximum, or the local maximum that aligns most closely to the ECG beat in time.

Embodiments of the computer-implemented method for generating an annotated PPG signal according to the invention, as defined by claim 8, further comprise:
- verifying if the PPG beat represents the highest local maximum within a time interval ranging from an ECG beat preceding said ECG beat in the ECG signal to an ECG beat following the ECG beat in the ECG signal; and
- associating the PPG beat with the ECG beat only when the PPG beat represents the highest local maximum.

Thus, in embodiments of the method according to the invention, a PPG beat is detected if it aligns with the ECG beat in the specified small time window, and if the PPG beat is the highest local maximum in the time interval between the previous ECG beat and the next ECG beat, i.e. the time interval [beat-1; beat+1] in the ECG signal. In that case, the ECG beat is mapped onto the detected PPG beat or - in other words - the ECG beat is paired with the detected PPG beat. In case another local maximum is detected in that interval with higher amplitude, for instance as a result of an artefact in the PPG measurement, no PPG beat is detected and no mapping of the ECG beat onto a PPG beat is possible. The fact that mapping of the ECG beat is possible or impossible will determine the annotation of a time-corresponding PPG segment. PPG signal segments for which no ECG beat can be mapped onto a PPG beat may be assumed bad quality segments and may therefore be annotated as "insufficient quality"; for PPG signal segments wherein an ECG beat can be mapped onto a PPG beat, the corresponding ECG annotation may be copied; for ECG signal portions where the quality is insufficient, no annotation may be copied onto the corresponding PPG signal segment or the corresponding PPG signal segment may be annotated as "undefined".

Embodiments of the computer-implemented method for generating an annotated PPG signal according to the invention, as defined by claim 9, further comprise:
- storing the annotated PPG signal in a database of training data used for training in machine learning.

In one example application, the annotated PPG signal obtained through the method according to the present invention, with derived bad quality portions, will be stored in a database to be used later as training data for machine learning or deep learning tools as used for instance in PPG signal classifiers.

Embodiments of the computer-implemented method for annotating a PPG signal according to the invention, as defined by claim 10, further comprise:
- training a neural network with said annotated PPG signal.

Thus, the annotated PPG signal obtained through the method according to the present invention and stored in a database of training data, may be used as training data for machine learning or deep learning tools as used for instance in PPG signal classifiers. A machine learning tool trained this way, shall be able to reliably detect various heart rhythms in a PPG signal (outcome = a medical diagnosis) as well as to identify bad quality portions in a PPG signal (outcome = bad quality). Consequently, machine learning tools obtained through embodiments of the present invention can inform a person that another measurement is needed before a reliable medical diagnosis can be made.

Embodiments of the computer-implemented method for generating an annotated PPG signal according to the invention, as defined by claim 11, further comprise:
- recording a PPG signal, similarly to recording the PPG signal;
- pre-processing the PPG signal differently to obtain plural PPG signal versions;
- annotating the plural PPG signal versions, similarly to annotating the PPG signal, thereby generating plural annotated PPG signals;
- comparing the plural annotated PPG signals and deriving an optimal pre-processing for PPG signals based on the amount of insufficient quality annotations in the annotated PPG signals.

Thus, in addition to or as an alternative for obtaining high-quality annotations for training a neural network / machine learning model, there is another application wherein PPG signals annotated according to an embodiment of the present invention bring advantages. Mapping the ECG beats and annotations on PPG beats and PPG signal segments for different PPG versions obtained through different PPG pre-processing techniques allows to compare the different PPG pre-processing techniques. The annotated PPG signal version with lowest amount of derived insufficient quality segments for example may be assumed to be pre-processed through the most optimal pre-processing technique.

In embodiments of the computer-implemented method for annotating a PPG signal according to the invention, as defined by claim 12, the annotations comprise one or more of the following:
- regular sine rhythm;
- insufficient quality;
- undefined;
- irregular rhythm annotations comprising:
   - atrial fibrillation;
   - atrial flutter;
   - supraventricular tachycardia;
   - ventricular tachycardia;
   - sinus tachycardia;
   - bradycardia;
   - ventricular fibrillation;
   - premature atrial contraction; and
   - premature contraction;
- other non-rhythm clinical annotations comprising:
   - sleep apnea.

Improved PPG annotation according to the present invention allows to detect a wider range of heart rhythm disorders whereas nowadays, PPG is typically used for AF detection only. In addition, the improved PPG annotation according to the invention allows to detect insufficient quality on the ECG, insufficient quality on the PPG, impossibility or insufficient quality in the mapping between ECG and PPG, as well as other non-rhythm clinical annotations, like for instance sleep apnea. The skilled person however shall appreciate that the above list of possible annotations is non-exhaustive. Other rhythm-related or non-rhythm-related clinical or non-clinical annotations may be used in various embodiments of the method according to the invention. One example is for instance the new annotation "invisible premature contraction" described here above.

According to a second aspect, the present invention relates to a controller as defined by claim 13, the controller comprising at least one processor and at least one memory including computer program code, the at least one memory and computer program code being configured to, with the at least one processor, cause the controller to perform:
- recording an electrocardiogram signal, abbreviated ECG signal;
- recording a photoplethysmography signal, abbreviated PPG signal, semi-synchronously with the recording of the ECG signal;
- annotating segments in the ECG signal either algorithm-based or expert-based, resulting in ECG segment annotations;
- time-aligning the PPG signal and the ECG signal;
- detecting cardiac beats in the ECG signal, named ECG beats;
- detecting cardiac beats in the PPG signal, named PPG beats;
- pairing the ECG beats with the PPG beats;
- deriving annotations for PPG signal segments based on the nature of the ECG segment annotations and on how the ECG beats can be paired with the PPG beats; and
- annotating the PPG signal segments using the annotations, thereby generating the annotated PPG signal.

According to a third aspect, the present invention relates to a computer program product as defined by claim 14, the computer program product comprising computer-executable instructions for causing a controller to perform at least the following:
- recording an electrocardiogram signal, abbreviated ECG signal;
- recording a photoplethysmography signal, abbreviated PPG signal, semi-synchronously with the recording of the ECG signal;
- annotating segments in the ECG signal either algorithm-based or expert-based, resulting in ECG segment annotations;
- time-aligning the PPG signal and the ECG signal;
- detecting cardiac beats in the ECG signal, named ECG beats;
- detecting cardiac beats in the PPG signal, named PPG beats;
- pairing the ECG beats with the PPG beats;
- deriving annotations for PPG signal segments based on the nature of the ECG segment annotations and on how the ECG beats can be paired with the PPG beats; and
- annotating the PPG signal segments using the annotations, thereby generating the annotated PPG signal.

According to a fourth aspect, the present invention relates to a computer readable storage medium as defined by claim 15, the computer readable storage medium comprising computer-executable instructions for performing the following steps when the program is run on a computer:
- recording an electrocardiogram signal, abbreviated ECG signal;
- recording a photoplethysmography signal, abbreviated PPG signal, semi-synchronously with the recording of the ECG signal;
- annotating segments in the ECG signal either algorithm-based or expert-based, resulting in ECG segment annotations;
- time-aligning the PPG signal and the ECG signal;
- detecting cardiac beats in the ECG signal, named ECG beats;
- detecting cardiac beats in the PPG signal, named PPG beats;
- pairing the ECG beats with the PPG beats;
- deriving annotations for PPG signal segments based on the nature of the ECG segment annotations and on how the ECG beats can be paired with the PPG beats; and
- annotating the PPG signal segments using the annotations, thereby generating the annotated PPG signal.

### Brief Description of the Drawings

Fig. 1 is a flow scheme illustrating an embodiment of the method for generating an annotated PPG signal according to the present invention;
Fig. 2A and 2B illustrate the steps 101-103 of recording an ECG signal, recording a PPG signal and annotating an ECG signal in an embodiment of the method for generating an annotated PPG signal according to the present invention;
Fig. 3A and 3B illustrate the steps 105-106 of detecting ECG beats and detecting PPG beats in an embodiment of the method for generating an annotated PPG signal according to the present invention;
Fig. 4A and 4B illustrate the steps 107-109 of mapping ECG beats onto PPG beats, deriving PPG annotations and annotating the PPG signal in an embodiment of the method for generating an annotated PPG signal according to the present invention;
Fig. 5A and 5B illustrate the steps 107-109 of mapping ECG beats onto PPG beats, deriving PPG annotations and annotating the PPG signal in a variant embodiment of the method for generating an annotated PPG signal according to the present invention;
Fig. 6A and 6B illustrate the steps 107-109 of mapping ECG beats onto PPG beats, deriving PPG annotations and annotating the PPG signal in another variant embodiment of the method for generating an annotated PPG signal according to the present invention;
Fig. 7A and 7B illustrate the steps 107-109 of mapping ECG beats onto PPG beats, deriving PPG annotations and annotating the PPG signal in yet another variant embodiment of the method for generating an annotated PPG signal according to the present invention; and
Fig. 8 illustrates a suitable computing system 800 for realizing embodiments of the method for PPG signal annotation in accordance with the present invention.

### Detailed Description of Embodiment(s)

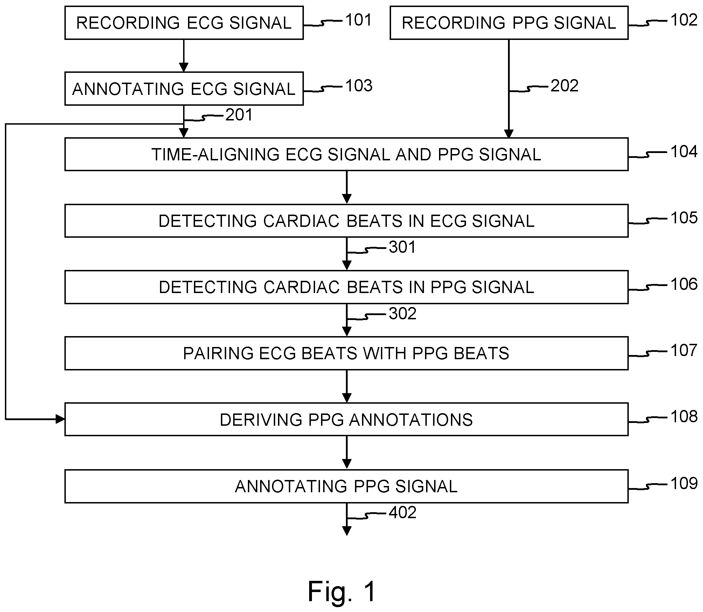

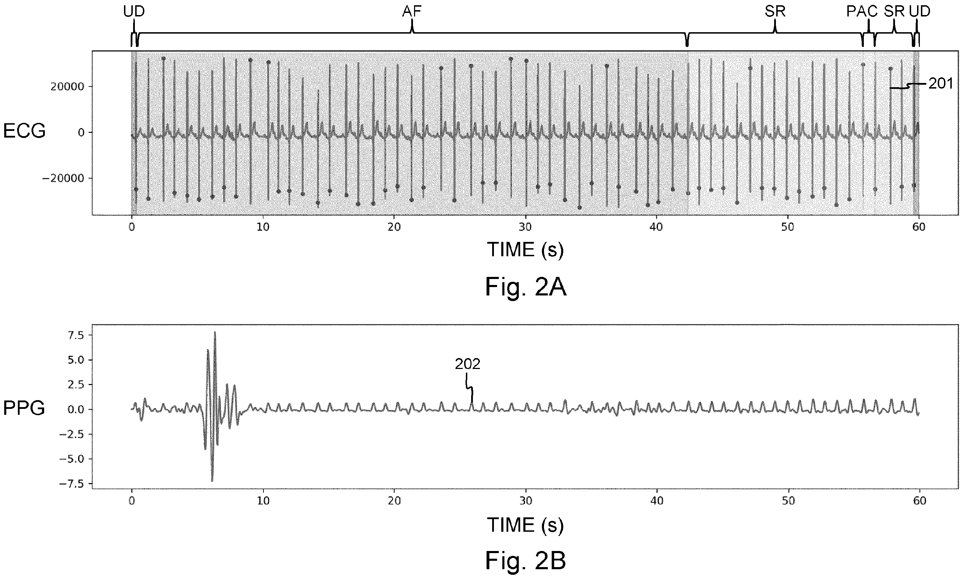

Fig. 1 shows the steps executed in an embodiment of the computer-implemented method for generating an annotated PPG signal 402 according to the present invention. In the steps 101 and 102, an ECG signal 201 and a PPG signal 202 are semi-synchronously recorded using different devices on a single person or animal. The ECG signal 201 is obtained by an ECG monitoring system, for example a Holter monitoring system. A 60 seconds long portion of the ECG signal 201 is shown in Fig. 2A. The PPG signal 202 is obtained by a PPG sensor, for example a pulse oximeter or smartphone camera. A 60 seconds long portion of the PPG signal 202 is shown in Fig. 2B. In step 103, the ECG signal 201 is annotated either algorithm-based or expert-based by one or plural people like for instance nurses, medical technicians, data scientists, cardiologists, etc. The annotating of the ECG signal 201 results in a first part of the 60 seconds portion shown in Fig. 2A being annotated as "Undefined" or "UD", a second part being annotated as "Atrial Fibrillation" or "AF", a third part being annotated as "Regular Sine Rhythm" or "SR", a fourth part being annotated as "Premature Atrial Contraction" or "PAC", a fifth part being annotated as "Regular Sine Rhythm" or "SR", and a sixth part being annotated as "Undefined" or "UD". The ECG signal 201 and PPG signal 202 that were semi-synchronously recorded are time-aligned in step 104. This means the ECG signal 201 and PPG signal 202 are synchronised in time with milliseconds accuracy by compensating for the offset in between the start times of the recordings of the ECG and PPG signals in steps 101 and 102, and by compensating for the drift between the clocks of the ECG monitoring system used in step 101 and the PPG sensor device used in step 102. It is noticed that the 60 seconds portions of the ECG signal 201 and PPG signal 202 shown in Fig. 2A and 2B are time-corresponding portions. Thus, it is assumed in Fig. 2A and Fig. 2B that the time-alignment of step 104 has already been executed.

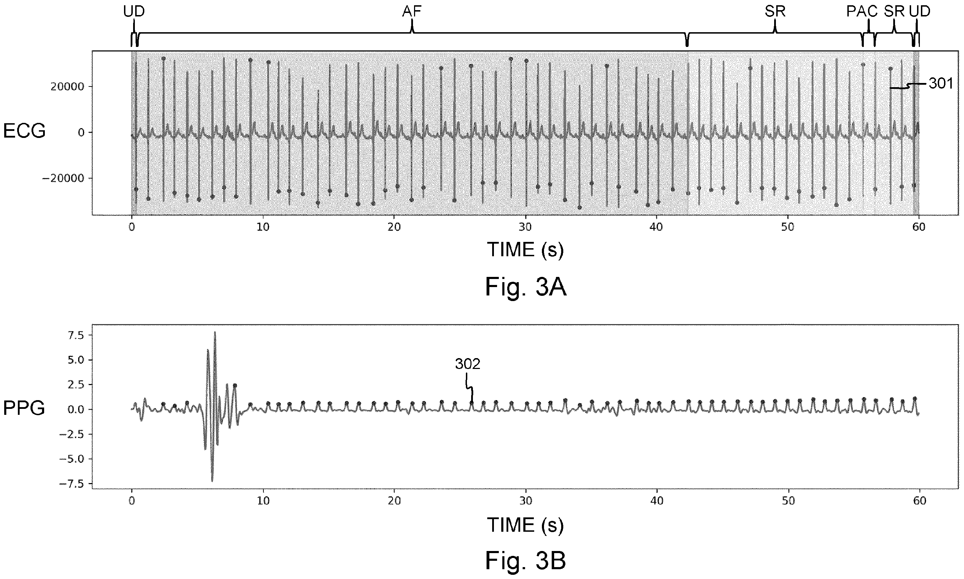

In steps 105-107, individual ECG beats are paired with PPG beats. In step 105, cardiac beats are identified in the ECG signal 201. The cardiac beats are shown in Fig. 3A wherein ECG signal 301 corresponds to ECG signal 201 and the detected cardiac beats are identified through the dots. It is noticed that the detection of cardiac beats in the ECG signal 201 may have taken place before or after the ECG signal was expert-based or algorithm based annotated in step 103, or may have taken place as part of this step 103. If for instance the ECG annotation in step 103 is executed on beat-to-beat segments, i.e. segments of variable length that range from one cardiac beat to the next cardiac beat, the cardiac beats must have been detected before the annotation in step 103 took place. If however the annotation in step 103 was done on fixed length segments of for instance 1 second, the cardiac beat detection for the ECG signal may be executed after the annotation of this signal in step 103. In step 106, for every ECG beat detected in step 105, a corresponding PPG beat is searched in the aligned PPG signal by looking for a local maximum in the PPG signal. This results in PPG signal 302 which corresponds to PPG signal 202 with detected beats identified by the dots. In an example embodiment of the invention, local maxima are detected in small, aligned time windows, in the PPG signal 302. As there might subsist some small misalignment between an ECG beat in ECG signal 301 and the corresponding PPG beat in PPG signal 302, the local maximum is searched for in a small window, for instance having a total length of 100 milliseconds (50 ms left and 50 ms right of the ECG beat). A PPG beat is detected if it aligns with the ECG beat in the specified small time window, and if the PPG beat is the highest local maximum in the time interval between the previous ECG beat and the next ECG beat, i.e. the interval [beat-1; beat+1] in the ECG signal 301. In such case, the detected PPG beat is mapped onto the time-corresponding ECG beat in step 107 or - in other words - the detected PPG beat is paired with the time-corresponding ECG beat. In case another local maximum is detected in that interval with higher amplitude, for instance as a result of an artefact in the PPG signal measurement, no PPG beat is detected and no pairing of the time-corresponding ECG beat is possible in step 107.

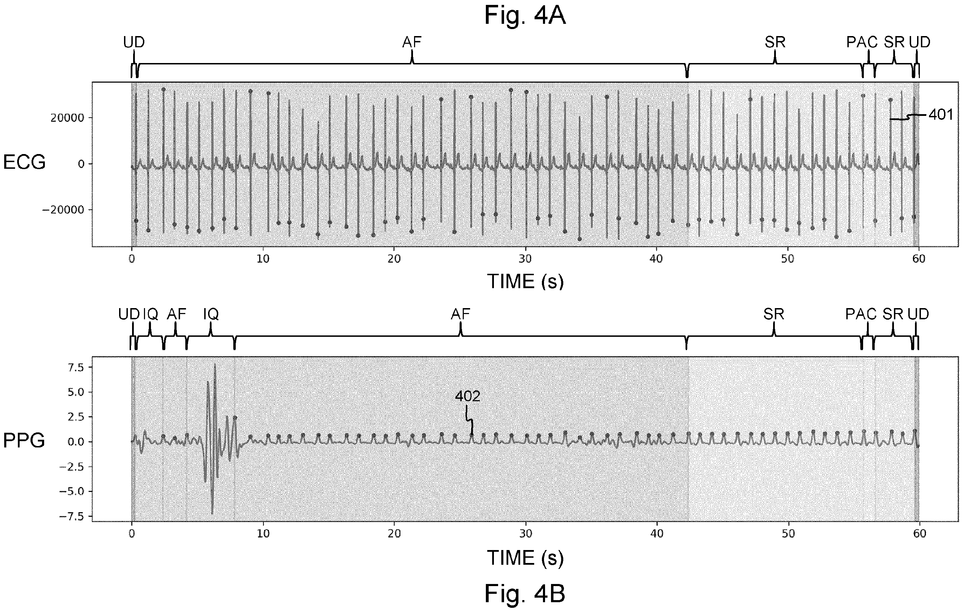

Once the ECG beats are paired with the PPG beats, the ECG annotations from step 103 (more precisely their nature) and information on how the ECG beats can be paired with the PPG beats in step 107 are used in step 108 to derive annotations for PPG signal segments and to annotate the PPG signal accordingly in step 109. PPG signal portions for which no ECG beat can be paired with a PPG beat in step 107, are assumed to be low quality portions in an embodiment of the invention illustrated by Fig. 4A and Fig. 4B. For such PPG signal portions, the annotation of the time-corresponding ECG signal portion is not copied. Instead, the PPG signal portion receives the annotation "Insufficient Quality" or "IQ". In Fig. 4B, two portions of the PPG signal 402 are annotated "IQ" because it is not possible for segments that form part of these PPG signal portions to pair an ECG beat with a PPG beat. For PPG signal portions wherein it is possible in step 107 to pair an ECG beat with a PPG beat, the annotation of the time-corresponding ECG segments is copied onto the PPG segments. In Fig. 4B, a first portion of the PPG signal 402 receives the annotation "UD" copied from the time-corresponding segments of the ECG signal 401, a third portion of the PPG signal 402 receives the annotation "AF" copied from the time-corresponding segments of the ECG signal 401, a fifth portion of the PPG signal 402 receives the annotation "AF" copied from the time-corresponding portion of the ECG signal 401, a sixth portion of the PPG signal 402 receives the annotation "SR" copied from the time-corresponding segments of the ECG signal 401, a seventh portion of the PPG signal 402 receives the annotation "PAC" copied from the time-corresponding segments of the ECG signal 401, an eight portion of the PPG signal 402 receives the annotation "SR" copied from the time-corresponding segments of the ECG signal 401, and a ninth portion of the PPG signal 402 receives the annotation "UD" copied from the time-corresponding segments of the ECG signal 401, because it was possible in all of these signal portions to pair each ECG beat with a PPG beat. It is noticed that the ECG signal 401 corresponds to the ECG signal 301 of Fig. 3A and ECG signal 201 of Fig. 2A.

The so obtained annotated PPG signal 402 with derived insufficient quality portions can be used as training data for a machine learning / deep learning tool that is used as PPG signal classifier. The machine learning tool trained this way shall be able to reliably detect various heart rhythms in a PPG signal - outcome of the PPG signal classifier is a medical diagnosis - and to identify insufficient quality portions in a PPG signal - outcome of the PPG signal classifier is "Insufficient Quality". Consequently, the machine learning tool obtained through such embodiment of the present invention can inform that another measurement is needed before a reliable medical diagnosis can be made.

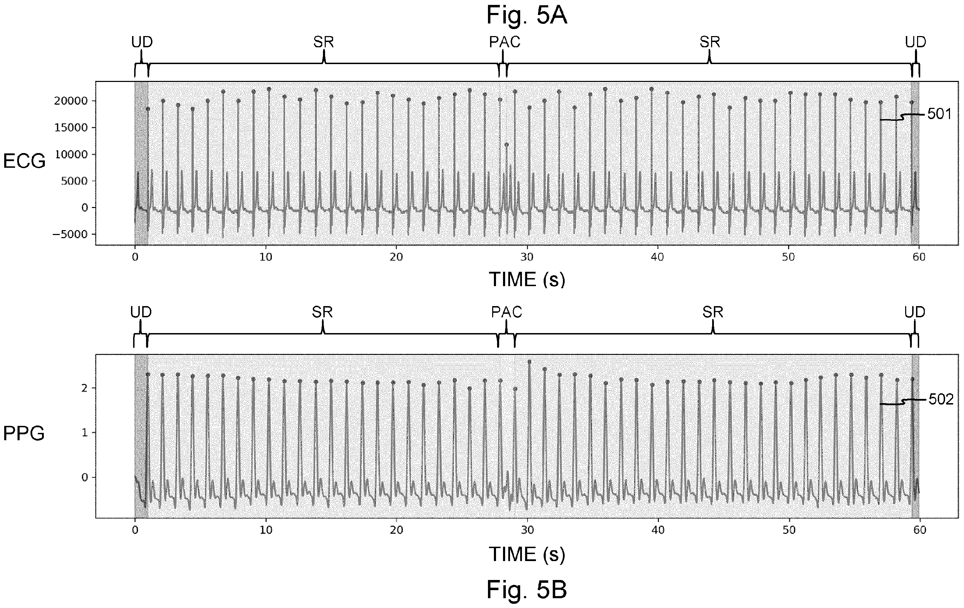

Fig. 5A and 5B illustrate a variant embodiment of the method for generating an annotated PPG signal according to the present invention. If in step 107, ECG beats cannot be paired with PPG beats, step 108 does not derive the annotation "Insufficient Quality" for the time-corresponding PPG sections but instead preserves the annotation of the time-corresponding ECG segments. Consequently, the annotation "UD" of a first portion of ECG signal 501 is copied onto a time-corresponding portion of PPG signal 502, the annotation "SR" of a second portion of ECG signal 501 is copied onto a time-corresponding portion of PPG signal 502, the annotation "PAC" of a third portion of ECG signal 501 is copied onto a time-corresponding portion of PPG signal 502, the annotation "SR" of a fourth portion of ECG signal 501 is copied onto a time-corresponding portion of PPG signal 502, and the annotation "UD" of a fifth portion of ECG signal 501 is copied onto a time-corresponding portion of PPG signal 502. Such an embodiment is desirable, for instance in situations wherein an ECG beat remains invisible in the PPG signal, for instance in case of a premature cardiac contraction which leads to low blood ejection from the heart. Rather than annotating the time-corresponding PPG section as "Insufficient Quality", it is desirable in such case to preserve the time-corresponding ECG segment annotation(s).

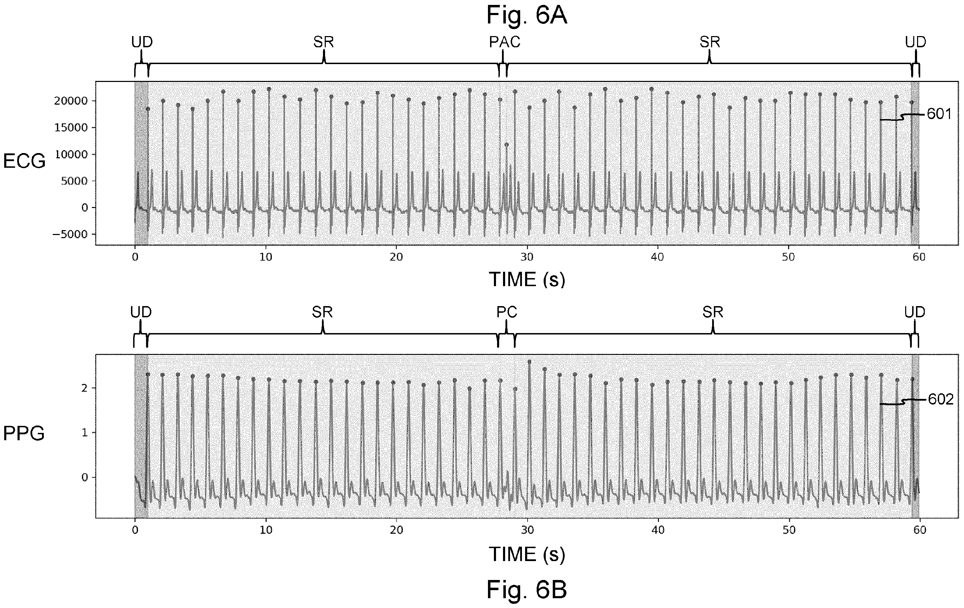

Fig. 6A and 6B illustrate another variant embodiment of the method for generating an annotated PPG signal according to the present invention. If in step 107, ECG beats cannot be paired with PPG beats, step 108 does not derive the annotation "Insufficient Quality" for the time-corresponding PPG sections but instead sets a new annotation for the time-corresponding ECG segments. Consequently, the annotation "UD" of a first portion of ECG signal 601 is copied onto a time-corresponding portion of PPG signal 602, the annotation "SR" of a second portion of ECG signal 601 is copied onto a time-corresponding portion of PPG signal 602, the new annotation "Premature Contraction" or "PC" is set for a third portion of the PPG signal 602 because ECG beats cannot be mapped onto PPG beats for the time-corresponding third portion of the ECG signal 601 with annotation "PAC", the annotation "SR" of a fourth portion of ECG signal 601 is copied onto a time-corresponding portion of PPG signal 602, and the annotation "UD" of a fifth portion of ECG signal 601 is copied onto a time-corresponding portion of PPG signal 602. Such an embodiment is desirable, for instance in situations wherein an ECG beat remains invisible in the PPG signal, for instance in case of a premature cardiac contraction which leads to low blood ejection from the heart. Rather than annotating the time-corresponding PPG section as "Insufficient Quality", it may be desirable in such case to attribute a new annotation, for example "Premature Contraction", derived from the nature of the time-corresponding ECG segment annotation.

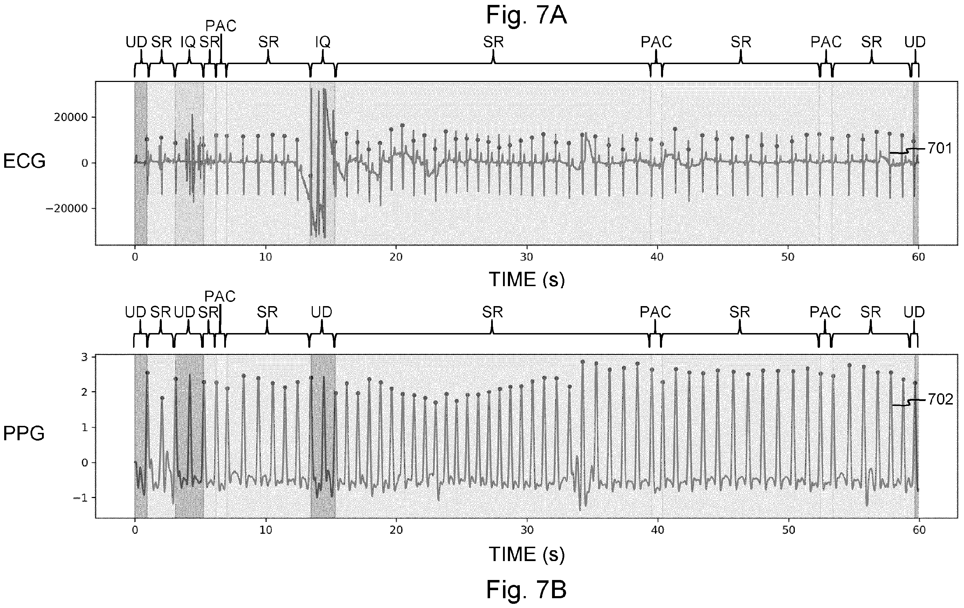

Fig. 7A and 7B illustrate yet another variant embodiment of the method for generating an annotated PPG signal according to the present invention. If in step 107, a section of the ECG signal 701 has insufficient quality, the time-corresponding PPG section is assigned the annotation "Undefined" or "UD" in step 108, because the PPG signal not necessarily has insufficient quality when the ECG signal has insufficient quality. Consequently, the annotation "UD" of a first portion of ECG signal 701 is copied onto a time-corresponding portion of PPG signal 702, the annotation "SR" of a second portion of ECG signal 701 is copied onto a time-corresponding portion of PPG signal 702, the annotation "UD" is assigned to a third portion of the PPG signal 702 because the time-corresponding portion of the ECG signal 701 has insufficient quality not allowing to pair ECG beats with PPG beats, the annotation "SR" of a fourth portion of ECG signal 701 is copied onto a time-corresponding portion of PPG signal 702, the annotation "PAC" of a fifth portion of ECG signal 701 is copied onto a time-corresponding portion of PPG signal 702, the annotation "SR" of a sixth portion of ECG signal 701 is copied onto a time-corresponding portion of PPG signal 702, the annotation "UD" is assigned to a seventh portion of the PPG signal 702 because the time-corresponding portion of the ECG signal 701 has insufficient quality not allowing to pair ECG beats with PPG beats, the annotation "SR" of a fourth portion of ECG signal 701 is copied onto a time-corresponding portion of PPG signal 702, the annotation "SR" of an eight portion of ECG signal 701 is copied onto a time-corresponding portion of PPG signal 702, the annotation "PAC" of a ninth portion of ECG signal 701 is copied onto a time-corresponding portion of PPG signal 702, the annotation "SR" of a tenth portion of ECG signal 701 is copied onto a time-corresponding portion of PPG signal 702, the annotation "PAC" of an eleventh portion of ECG signal 701 is copied onto a time-corresponding portion of PPG signal 702, the annotation "SR" of a twelfth portion of ECG signal 701 is copied onto a time-corresponding portion of PPG signal 702, and the annotation "UD" of a thirteenth portion of ECG signal 701 is copied onto a time-corresponding portion of PPG signal 702.

The skilled person will appreciate that several of the rules for deriving annotations for PPG signal segments based on the nature of time-corresponding ECG signal annotations and on how the pairing of ECG beats onto PPG beats can be done as described here above for the embodiments illustrated by Fig. 4A-4B, Fig. 5A-5B, Fig. 6A-6B and/or Fig. 7A-7B can be combined with each other to generate further variant embodiments of the method for PPG signal annotation according to the present invention.

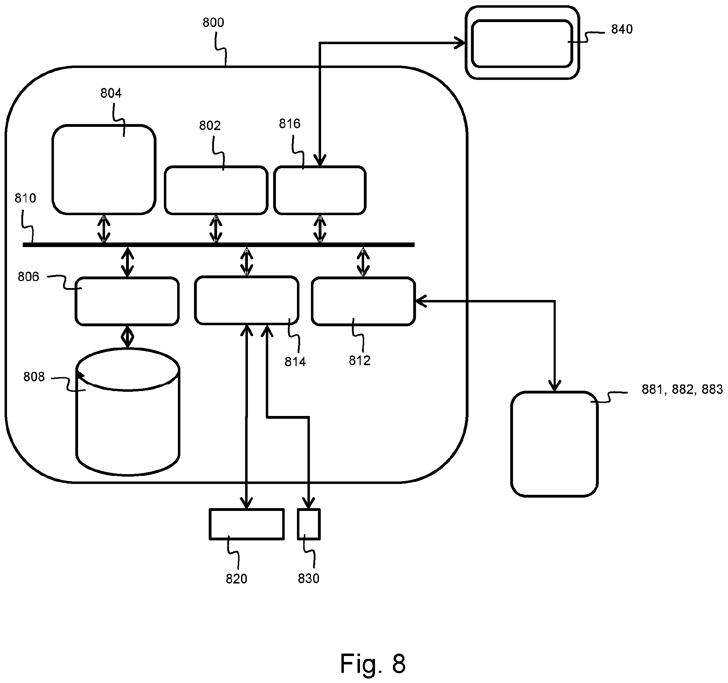

Fig. 8 shows a suitable computing system 800 according to an embodiment of the invention. Computing system 800 is suitable for implementing embodiments of the method for PPG signal annotation in line with the present invention. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806 and one or more storage element 808. Bus 810 may comprise one or more conductors that permit communication among the components of the computing system 800. Processor 802 may include any type of conventional processor or microprocessor that interprets and executes programming instructions. Local memory 804 may include a random access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanism that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage elements 808 above is described as a local disk, in general any other suitable computer-readable media such as a removable magnetic disk, optical storage media such as a CD or DVD, -ROM disk, solid state drives, flash memory cards, ... could be used. It is noticed that the entire method according to the present invention can be executed centralized, e.g. on a server in a management centre or in a cloud system, or it can be partially executed on a remote electronic device, e.g. worn by the user, and partially on a central server. Computing system 800 could thus correspond to the processing system available centrally or the processing system available in the electronic device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for generating an annotated photoplethysmography signal (402), abbreviated annotated PPG signal, said method comprising:
- recording (101) an electrocardiogram signal (201), abbreviated ECG signal, using an ECG monitoring system;
- recording (102) a photoplethysmography signal (202), abbreviated PPG signal, semi-synchronously with said recording (101) of said ECG signal (201), using a contact PPG sensor;
- annotating (103) segments in said ECG signal (201) either algorithm-based or expert-based, resulting in ECG segment annotations;
- time-aligning (104) said PPG signal (202) and said ECG signal (201);
- detecting (105) cardiac beats in said ECG signal (201), named ECG beats (301);
- detecting (106) cardiac beats in said PPG signal (202), named PPG beats (302);
- pairing (107) said ECG beats (301) with said PPG beats (302);
- deriving (108) annotations for PPG signal segments based on the nature of said ECG segment annotations and on how said ECG beats (301) can be paired with said PPG beats (302); and
- annotating (109) said PPG signal segments using said annotations, thereby generating said annotated PPG signal (402).

2. A computer-implemented method for generating an annotated PPG signal (402; 502; 602; 702) according to claim 1, wherein deriving annotations for PPG signal segments comprises:
- copying an annotation of an ECG segment onto a time-corresponding PPG segment when an ECG beat can be paired with a PPG beat for said segment.

3. A computer-implemented method for generating an annotated PPG signal (402; 702) according to claim 1, wherein deriving annotations for PPG signal segments comprises:
- marking a PPG segment as insufficient quality segment when no ECG beat can be paired with a PPG beat for said segment or when no PPG beat can be paired with an ECG beat for said segment.

4. A computer-implemented method for generating an annotated PPG signal (502) according to claim 1, wherein deriving annotations for PPG signal segments comprises:
- copying an annotation of an ECG segment onto a time-corresponding PPG segment when an ECG beat cannot be paired with a PPG beat for said segment or attributing a new annotation to a time-corresponding PPG segment when an ECG beat cannot be paired with a PPG beat for said segment.

5. A computer-implemented method for generating an annotated PPG signal (702) according to claim 1, wherein deriving annotations for PPG signal segments comprises:
- marking a PPG segment as undefined segment when no ECG beat can be paired with a PPG beat for said segment because a time-corresponding ECG segment has insufficient quality.

6. A computer-implemented method for generating an annotated PPG signal (402) according to one of the preceding claims, wherein pairing (107) said ECG beats (301) with said PPG beats (302) comprises:
- selecting an ECG beat;
- determining an ECG signal portion comprising said ECG beat and no other ECG beats;
- determining a PPG signal portion time-aligned with said ECG signal portion;
- identifying a PPG beat lying within said PPG signal portion;
- associating said PPG beat with said ECG beat.

7. A computer-implemented method for generating an annotated PPG signal (402) according to one of the preceding claims, wherein detecting (106) said PPG beats (302) comprises detecting local maxima in said PPG signal.

8. A computer-implemented method for generating an annotated PPG signal (402) according to claim 6 and claim 7, further comprising:
- verifying if said PPG beat represents the highest local maximum within a time interval ranging from an ECG beat preceding said ECG beat in said ECG signal to an ECG beat following said ECG beat in said ECG signal; and
- associating said PPG beat with said ECG beat only when said PPG beat represents the highest local maximum.

9. A computer-implemented method for generating an annotated PPG signal (402) according to one of the preceding claims, further comprising:
- storing said annotated PPG signal (402) in a database of training data used for training in machine learning.

10. A computer-implemented method for generating an annotated PPG signal (402) according to claim 5, further comprising:
- training a neural network with said annotated PPG signal (402).

11. A computer-implemented method for generating an annotated PPG signal (402) according to one of the preceding claims, further comprising:
- recording a PPG signal, similarly to recording said PPG signal (202);
- pre-processing said PPG signal differently to obtain plural PPG signal versions;
- annotating said plural PPG signal versions, similarly to annotating said PPG signal, thereby generating plural annotated PPG signals;
- comparing said plural annotated PPG signals and deriving an optimal pre-processing for PPG signals based on the amount of insufficient quality annotations in said annotated PPG signals.

12. A computer-implemented method for generating an annotated PPG signal (402) according to one of the preceding claims, wherein said annotations comprise one or more of the following:
- regular sine rhythm (SR);
- insufficient quality (IQ);
- undefined (UD);
- irregular rhythm annotations comprising:
- atrial fibrillation (AF);
- atrial flutter;
- supraventricular tachycardia;
- ventricular tachycardia;
- sinus tachycardia;
- bradycardia;
- ventricular fibrillation;
- premature atrial contraction (PAC); and
- premature contraction (PC);
- other non-rhythm clinical annotations comprising:
- sleep apnea.

13. A controller (800) comprising at least one processor (802) and at least one memory (804) including computer program code, the at least one memory (804) and computer program code being configured to, with the at least one processor (802), cause the controller (800) to perform:
- recording (101) an electrocardiogram signal (201), abbreviated ECG signal;
- recording (102) a photoplethysmography signal (202), abbreviated PPG signal, semi-synchronously with said recording (101) of said ECG signal;
- annotating (103) segments in said ECG signal (201) either algorithm-based or expert-based, resulting in ECG segment annotations;
- time-aligning (104) said PPG signal (202) and said ECG signal (201);
- detecting (105) cardiac beats (301) in said ECG signal, named ECG beats;
- detecting (106) cardiac beats (302) in said PPG signal, named PPG beats;
- pairing (107) said ECG beats (301) with said PPG beats (302);
- deriving (108) annotations for PPG signal segments based on the nature of said ECG segment annotations and on how said ECG beats (301) can be paired with said PPG beats (302); and
- annotating (109) said PPG signal segments using said annotations, thereby generating said annotated PPG signal (402).

14. A computer program product comprising computer-executable instructions for causing a controller (800) to perform at least the following:
- recording (101) an electrocardiogram signal (201), abbreviated ECG signal;
- recording (102) a photoplethysmography signal (202), abbreviated PPG signal, semi-synchronously with said recording (101) of said ECG signal;
- annotating (103) segments in said ECG signal (201) either algorithm-based or expert-based, resulting in ECG segment annotations;
- time-aligning (104) said PPG signal (202) and said ECG signal (201);
- detecting (105) cardiac beats (301) in said ECG signal, named ECG beats;
- detecting (106) cardiac beats (302) in said PPG signal, named PPG beats;
- pairing (107) said ECG beats (301) with said PPG beats (302);
- deriving (108) annotations for PPG signal segments based on the nature of said ECG segment annotations and on how said ECG beats (301) can be paired with said PPG beats (302); and
- annotating (109) said PPG signal segments using said annotations, thereby generating said annotated PPG signal (402).

15. A computer readable storage medium comprising computer-executable instructions of a program for performing the following steps when the program is run on a computer (800):
- recording (101) an electrocardiogram signal (201), abbreviated ECG signal;
- recording (102) a photoplethysmography signal (202), abbreviated PPG signal, semi-synchronously with said recording (101) of said ECG signal;
- annotating (103) segments in said ECG signal (201) either algorithm-based or expert-based, resulting in ECG segment annotations;
- time-aligning (104) said PPG signal (202) and said ECG signal (201);
- detecting (105) cardiac beats (301) in said ECG signal, named ECG beats;
- detecting (106) cardiac beats (302) in said PPG signal, named PPG beats;
- pairing (107) said ECG beats (301) with said PPG beats (302);
- deriving (108) annotations for PPG signal segments based on the nature of said ECG segment annotations and on how said ECG beats (301) can be paired with said PPG beats (302); and
- annotating (109) said PPG signal segments using said annotations, thereby generating said annotated PPG signal (402).

## Patentansprüche

1. Computerimplementiertes Verfahren zum Erzeugen eines kommentierten Photoplethysmographiesignals (402), abgekürzt kommentiertes PPG-Signal, wobei das Verfahren Folgendes umfasst:
- Aufzeichnen (101) eines Elektrokardiogrammsignals (201), abgekürzt EKG-Signal, unter Verwendung eines EKG-Überwachungssystems;
- Aufzeichnen (102) eines Photoplethysmographiesignals (202), abgekürzt PPG-Signal, halbsynchron mit dem Aufzeichnen (101) des EKG-Signals (201) unter Verwendung eines PPG-Kontaktsensors;
- Kommentieren (103) von Segmenten in dem EKG-Signal (201) entweder algorithmusbasiert oder expertenbasiert, was zu EKG-Segment-Kommentaren führt;
- zeitliches Ausrichten (104) des PPG-Signals (202) und des EKG-Signals (201);
- Erfassen (105) von Herzschlägen in dem EKG-Signal (201), genannt EKG-Schläge (301);
- Erfassen (106) von Herzschlägen in dem PPG-Signal (202), genannt PPG-Schläge (302);
- Paaren (107) der EKG-Schläge (301) mit den PPG-Schlägen (302);
- Ableiten (108) von Kommentaren für PPG-Signalsegmente basierend auf der Natur der EKG-Segmentkommentare und darauf, wie die EKG-Schläge (301) mit den PPG-Schlägen (302) gepaart werden können; und
- Kommentieren (109) der PPG-Signalsegmente unter Verwendung der Kommentare, wodurch das kommentierte PPG-Signal (402) erzeugt wird.

2. Computerimplementiertes Verfahren zum Erzeugen eines kommentierten PPG-Signals (402; 502; 602; 702) nach Anspruch 1, wobei das Ableiten von Kommentaren für PPG-Signalsegmente Folgendes umfasst:
- Kopieren eines Kommentars eines EKG-Segments auf ein zeitlich entsprechendes PPG-Segment, wenn ein EKG-Schlag mit einem PPG-Schlag für das Segment gepaart werden kann.

3. Computerimplementiertes Verfahren zum Erzeugen eines kommentierten PPG-Signals (402; 702) nach Anspruch 1, wobei das Ableiten von Kommentaren für PPG-Signalsegmente Folgendes umfasst:
- Markieren eines PPG-Segments als Segment mit unzureichender Qualität, wenn kein EKG-Schlag mit einem PPG-Schlag für das Segment gepaart werden kann oder wenn kein PPG-Schlag mit einem EKG-Schlag für das Segment gepaart werden kann.

4. Computerimplementiertes Verfahren zum Erzeugen eines kommentierten PPG-Signals (502) nach Anspruch 1, wobei das Ableiten von Kommentaren für PPG-Signalsegmente Folgendes umfasst:
- Kopieren eines Kommentars eines EKG-Segments auf ein zeitlich entsprechendes PPG-Segment, wenn ein EKG-Schlag nicht mit einem PPG-Schlag für das Segment gepaart werden kann, oder Zuweisen eines neuen Kommentars zu einem zeitlich entsprechenden PPG-Segment, wenn ein EKG-Schlag nicht mit einem PPG-Schlag für das Segment gepaart werden kann.

5. Computerimplementiertes Verfahren zum Erzeugen eines kommentierten PPG-Signals (702) nach Anspruch 1, wobei das Ableiten von Kommentaren für PPG-Signalsegmente Folgendes umfasst:
- Markieren eines PPG-Segments als undefiniertes Segment, wenn kein EKG-Schlag mit einem PPG-Schlag für das Segment gepaart werden kann, weil ein zeitlich entsprechendes EKG-Segment unzureichende Qualität aufweist.

6. Computerimplementiertes Verfahren zum Erzeugen eines kommentierten PPG-Signals (402) nach einem der vorhergehenden Ansprüche, wobei das Paaren (107) der EKG-Schläge (301) mit den PPG-Schlägen (302) Folgendes umfasst:
- Auswählen eines EKG-Schlags;
- Bestimmen eines EKG-Signalabschnittes, der den EKG-Schlag und keine anderen EKG-Schläge umfasst;
- Bestimmen eines PPG-Signalabschnittes, der zeitlich auf den EKG-Signalabschnitt ausgerichtet ist;
- Identifizieren eines PPG-Schlags, der innerhalb des PPG-Signalabschnittes liegt;
- Zuordnen des PPG-Schlags zu dem EKG-Schlag.

7. Computerimplementiertes Verfahren zum Erzeugen eines kommentierten PPG-Signals (402) nach einem der vorhergehenden Ansprüche, wobei das Erfassen (106) der PPG-Schläge (302) das Erfassen von lokalen Maxima in dem PPG-Signal umfasst.

8. Computerimplementiertes Verfahren zum Erzeugen eines kommentierten PPG-Signals (402) nach Anspruch 6 und Anspruch 7, ferner umfassend:
- Verifizieren, ob der PPG-Schlag das höchste lokale Maximum innerhalb eines Zeitintervalls darstellt, das von einem EKG-Schlag vor dem EKG-Schlag in dem EKG-Signal zu einem EKG-Schlag nach dem EKG-Schlag in dem EKG-Signal reicht; und
- Zuordnen des PPG-Schlags zu dem EKG-Schlag nur, wenn der PPG-Schlag das höchste lokale Maximum darstellt.

9. Computerimplementiertes Verfahren zum Erzeugen eines kommentierten PPG-Signals (402) nach einem der vorhergehenden Ansprüche, ferner umfassend:
- Speichern des kommentierten PPG-Signals (402) in einer Datenbank von Trainingsdaten, die zum Trainieren im maschinellen Lernen verwendet werden.

10. Computerimplementiertes Verfahren zum Erzeugen eines kommentierten PPG-Signals (402) nach Anspruch 5, ferner umfassend:
- Trainieren eines neuronalen Netzwerks mit dem kommentierten PPG-Signal (402).

11. Computerimplementiertes Verfahren zum Erzeugen eines kommentierten PPG-Signals (402) nach einem der vorhergehenden Ansprüche, ferner umfassend:
- Aufzeichnen eines PPG-Signals ähnlich dem Aufzeichnen des PPG-Signals (202);
- unterschiedliches Vorverarbeiten des PPG-Signals, um mehrere PPG-Signalversionen zu erhalten;
- Kommentieren der mehreren PPG-Signalversionen ähnlich dem Kommentieren des PPG-Signals, wodurch mehrere kommentierte PPG-Signale erzeugt werden;
- Vergleichen der mehreren kommentierten PPG-Signale und Ableiten einer optimalen Vorverarbeitung für PPG-Signale basierend auf der Menge an Kommentaren mit unzureichender Qualität in den kommentierten PPG-Signalen.

12. Computerimplementiertes Verfahren zum Erzeugen eines kommentierten PPG-Signals (402) nach einem der vorhergehenden Ansprüche, wobei die Kommentare eines oder mehrere des Folgenden umfassen:
- regelmäßigen Sinusrhythmus (SR);
- unzureichende Qualität (IQ);
- undefiniert (UD);
- Kommentare zu unregelmäßigem Rhythmus, umfassend:
- Vorhofflimmern (AF);
- Vorhofflattern:
- supraventrikuläre Tachykardie;
- ventrikuläre Tachykardie;
- Sinus-Tachykardie:
- Bradykardie;
- Kammerflimmern;
- vorzeitige atriale Kontraktion (PAC); und
- vorzeitige Kontraktion (PC);
- andere klinische Nicht-Rhythmus-Kommentare, umfassend:
- Schlafapnoe.

13. Steuerung (800), die zumindest einen Prozessor (802) und zumindest einen Speicher (804) umfasst, der Computerprogrammcode beinhaltet, wobei der zumindest eine Speicher (804) und der Computerprogrammcode konfiguriert sind, um mit dem zumindest einen Prozessor (802) die Steuerung (800) zu veranlassen, Folgendes durchzuführen:
- Aufzeichnen (101) eines Elektrokardiogrammsignals (201), abgekürzt EKG-Signal;
- Aufzeichnen (102) eines Photoplethysmographiesignals (202), abgekürzt PPG-Signal, halbsynchron mit dem Aufzeichnen (101) des EKG-Signals;
- Kommentieren (103) von Segmenten in dem EKG-Signal (201) entweder algorithmusbasiert oder expertenbasiert, was zu EKG-Segmentkommentaren führt;
- zeitliches Ausrichten (104) des PPG-Signals (202) und des EKG-Signals (201);
- Erfassen (105) von Herzschlägen (301) in dem EKG-Signal, genannt EKG-Schläge;
- Erfassen (106) von Herzschlägen (302) in dem PPG-Signal, genannt PPG-Schläge;
- Paaren (107) der EKG-Schläge (301) mit den PPG-Schlägen (302);
- Ableiten (108) von Kommentaren für PPG-Signalsegmente basierend auf der Natur der EKG-Segmentkommentare und darauf, wie die EKG-Schläge (301) mit den PPG-Schlägen (302) gepaart werden können; und
- Kommentieren (109) der PPG-Signalsegmente unter Verwendung der Kommentare, wodurch das kommentierte PPG-Signal (402) erzeugt wird.

14. Computerprogrammprodukt, das computerausführbare Anweisungen umfasst, um eine Steuerung (800) zu veranlassen, zumindest das Folgende durchzuführen:
- Aufzeichnen (101) eines Elektrokardiogrammsignals (201), abgekürzt EKG-Signal;
- Aufzeichnen (102) eines Photoplethysmographiesignals (202), abgekürzt PPG-Signal, halbsynchron mit dem Aufzeichnen (101) des EKG-Signals;
- Kommentieren (103) von Segmenten in dem EKG-Signal (201) entweder algorithmusbasiert oder expertenbasiert, was zu EKG-Segmentkommentaren führt;
- zeitliches Ausrichten (104) des PPG-Signals (202) und des EKG-Signals (201);
- Erfassen (105) von Herzschlägen (301) in dem EKG-Signal, genannt EKG-Schläge;
- Erfassen (106) von Herzschlägen (302) in dem PPG-Signal, genannt PPG-Schläge;
- Paaren (107) der EKG-Schläge (301) mit den PPG-Schlägen (302);
- Ableiten (108) von Kommentaren für PPG-Signalsegmente basierend auf der Natur der EKG-Segmentkommentare und darauf, wie die EKG-Schläge (301) mit den PPG-Schlägen (302) gepaart werden können; und
- Kommentieren (109) der PPG-Signalsegmente unter Verwendung der Kommentare, wodurch das kommentierte PPG-Signal (402) erzeugt wird.

15. Computerlesbares Speichermedium, das computerausführbare Anweisungen eines Programms zum Durchführen der folgenden Schritte umfasst, wenn das Programm auf einem Computer (800) läuft:
- Aufzeichnen (101) eines Elektrokardiogrammsignals (201), abgekürzt EKG-Signal;
- Aufzeichnen (102) eines Photoplethysmographiesignals (202), abgekürzt PPG-Signal, halbsynchron mit dem Aufzeichnen (101) des EKG-Signals;
- Kommentieren (103) von Segmenten in dem EKG-Signal (201) entweder algorithmusbasiert oder expertenbasiert, was zu EKG-Segmentkommentaren führt;
- zeitliches Ausrichten (104) des PPG-Signals (202) und des EKG-Signals (201);
- Erfassen (105) von Herzschlägen (301) in dem EKG-Signal, genannt EKG-Schläge;
- Erfassen (106) von Herzschlägen (302) in dem PPG-Signal, genannt PPG-Schläge;
- Paaren (107) der EKG-Schläge (301) mit den PPG-Schlägen (302);
- Ableiten (108) von Kommentaren für PPG-Signalsegmente basierend auf der Natur der EKG-Segmentkommentare und darauf, wie die EKG-Schläge (301) mit den PPG-Schlägen (302) gepaart werden können; und
- Kommentieren (109) der PPG-Signalsegmente unter Verwendung der Kommentare, wodurch das kommentierte PPG-Signal (402) erzeugt wird.

## Revendications

1. Procédé mis en œuvre par ordinateur permettant de générer un signal de photopléthysmographie annoté (402), en abrégé signal PPG annoté, ledit procédé comprenant :
- l'enregistrement (101) d'un signal d'électrocardiogramme (201), en abrégé signal ECG, à l'aide d'un système de surveillance d'ECG ;
- l'enregistrement (102) d'un signal de photopléthysmographie (202), en abrégé signal PPG, de manière semi-synchrone avec ledit enregistrement (101) dudit signal ECG (201), à l'aide d'un capteur de PPG à contact ;
- l'annotion (103) de segments dans ledit signal ECG (201) soit sur la base d'un algorithme, soit sur la base d'un expert, qui conduit à des annotations de segment d'ECG ;
- l'alignement temporel (104) dudit signal PPG (202) et dudit signal ECG (201) ;
- la détection (105) de battements cardiaques dans ledit signal ECG (201), appelés battements ECG (301) ;
- la détection (106) de battements cardiaques dans ledit signal PPG (202), appelés battements PPG (302) ;
- l'appariement (107) desdits battements ECG (301) auxdits battements PPG (302) ;
- la dérivation (108) d'annotations pour des segments de signal PPG en fonction de la nature desdites annotations de segment d'ECG et de la façon dont lesdits battements ECG (301) peuvent être appariés auxdits battements PPG (302) ; et
- l'annotation (109) desdits segments de signal PPG à l'aide desdites annotations, ce qui génère ledit signal PPG annoté (402).

2. Procédé mis en œuvre par ordinateur permettant de générer un signal PPG annoté (402 ; 502 ; 602 ; 702) selon la revendication 1, ladite dérivation d'annotations pour des segments de signal PPG comprenant :
- la copie d'une annotation d'un segment d'ECG sur un segment de PPG correspondant dans le temps lorsqu'un battement ECG peut être apparié à un battement PPG pour ledit segment.

3. Procédé mis en œuvre par ordinateur permettant de générer un signal PPG annoté (402 ; 702) selon la revendication 1, ladite dérivation d'annotations pour des segments de signal PPG comprenant :
- le marquage d'un segment de PPG comme segment de qualité insuffisante lorsqu'aucun battement ECG ne peut être apparié à un battement PPG pour ledit segment ou lorsqu'aucun battement PPG ne peut être apparié à un battement ECG pour ledit segment.

4. Procédé mis en œuvre par ordinateur permettant de générer un signal PPG annoté (502) selon la revendication 1, ladite dérivation d'annotations pour des segments de signal PPG comprenant :
- la copie d'une annotation d'un segment d'ECG sur un segment de PPG correspondant dans le temps lorsqu'un battement ECG ne peut pas être apparié à un battement PPG pour ledit segment ou l'attribution d'une nouvelle annotation à un segment de PPG correspondant dans le temps lorsqu'un battement ECG ne peut pas être apparié à un battement PPG pour ledit segment.

5. Procédé mis en œuvre par ordinateur permettant de générer un signal PPG annoté (702) selon la revendication 1, ladite dérivation d'annotations pour des segments de signal PPG comprenant :
- le marquage d'un segment de PPG comme segment non défini lorsqu'aucun battement ECG ne peut être apparié à un battement PPG pour ledit segment parce qu'un segment d'ECG correspondant dans le temps présente une qualité insuffisante.

6. Procédé mis en œuvre par ordinateur permettant de générer un signal PPG annoté (402) selon l'une des revendications précédentes, ledit appariement (107) desdits battements ECG (301) auxdits battements PPG (302) comprenant :
- la sélection d'un battement ECG ;
- la détermination d'une partie de signal ECG comprenant ledit battement ECG et aucun autre battement ECG ;
- la détermination d'une partie de signal PPG alignée dans le temps avec ladite partie de signal ECG ;
- l'identification d'un battement PPG se trouvant dans ladite partie de signal PPG ;
- l'association dudit battement PPG audit battement ECG.

7. Procédé mis en œuvre par ordinateur permettant de générer un signal PPG annoté (402) selon l'une des revendications précédentes, ladite détection (106) desdits battements PPG (302) comprenant la détection de maximums locaux dans ledit signal PPG.

8. Procédé mis en œuvre par ordinateur permettant de générer un signal PPG annoté (402) selon la revendication 6 et la revendication 7, comprenant en outre :
- la vérification pour savoir si ledit battement PPG représente le maximum local le plus élevé dans un intervalle de temps allant d'un battement ECG précédant ledit battement ECG dans ledit signal ECG à un battement ECG suivant ledit battement ECG dans ledit signal ECG ; et
- l'association dudit battement PPG audit battement ECG uniquement lorsque ledit battement PPG représente le maximum local le plus élevé.

9. Procédé mis en œuvre par ordinateur permettant de générer un signal PPG annoté (402) selon l'une des revendications précédentes, comprenant en outre :
- le stockage dudit signal PPG annoté (402) dans une base de données de données de formation utilisées pour la formation en apprentissage automatique.

10. Procédé mis en œuvre par ordinateur permettant de générer un signal PPG annoté (402) selon la revendication 5, comprenant en outre :
- la formation d'un réseau neuronal avec ledit signal PPG annoté (402).

11. Procédé mis en œuvre par ordinateur permettant de générer un signal PPG annoté (402) selon l'une des revendications précédentes, comprenant en outre :
- l'enregistrement d'un signal PPG, de manière similaire à l'enregistrement dudit signal PPG (202) ;
- le prétraitement dudit signal PPG différemment pour obtenir plusieurs versions de signal PPG ;
- l'annotation desdites plusieurs versions de signal PPG, de manière similaire à l'annotation dudit signal PPG, ce qui génère plusieurs signaux PPG annotés ;
- la comparaison desdits plusieurs signaux PPG annotés et la dérivation d'un prétraitement optimal pour les signaux PPG sur la base de la quantité d'annotations de qualité insuffisante dans lesdits signaux PPG annotés.

12. Procédé mis en œuvre par ordinateur permettant de générer un signal PPG annoté (402) selon l'une des revendications précédentes, lesdites annotations comprenant un ou plusieurs des éléments suivants :
- un rythme sinusoïdal régulier (SR) ;
- une qualité insuffisante (IQ) ;
- non défini (UD) ;
- des annotations de rythme irrégulier comprenant :
- une fibrillation auriculaire (AF) ;
- un flutter auriculaire ;
- une tachycardie supraventriculaire ;
- une tachycardie ventriculaire ;
- une tachycardie sinusale ;
- une bradycardie ;
- une fibrillation ventriculaire ;
- une contraction auriculaire prématurée (PAC) ; et
- une contraction prématurée (PC) ;
- d'autres annotations cliniques non rythmiques comprenant :
- l'apnée du sommeil.

13. Dispositif de commande (800) comprenant au moins un processeur (802) et au moins une mémoire (804) comprenant un code de programme informatique, ladite au moins une mémoire (804) et ledit code de programme informatique étant configurés pour, avec ledit au moins un processeur (802), amener le dispositif de commande (800) à effectuer :
- l'enregistrement (101) d'un signal d'électrocardiogramme (201), en abrégé signal ECG ;
- l'enregistrement (102) d'un signal de photopléthysmographie (202), en abrégé signal PPG, de manière semi-synchrone avec ledit enregistrement (101) dudit signal ECG ;
- l'annotion (103) de segments dans ledit signal ECG (201) soit sur la base d'un algorithme, soit sur la base d'un expert, qui conduit à des annotations de segment d'ECG ;
- l'alignement temporel (104) dudit signal PPG (202) et dudit signal ECG (201) ;
- la détection (105) de battements cardiaques (301) dans ledit signal ECG, appelés battements ECG ;
- la détection (106) de battements cardiaques (302) dans ledit signal PPG, appelés battements PPG ;
- l'appariement (107) desdits battements ECG (301) auxdits battements PPG (302) ;
- la dérivation (108) d'annotations pour des segments de signal PPG en fonction de la nature desdites annotations de segment d'ECG et de la façon dont lesdits battements ECG (301) peuvent être appariés auxdits battements PPG (302) ; et
- l'annotation (109) desdits segments de signal PPG à l'aide desdites annotations, ce qui génère ledit signal PPG annoté (402).

14. Produit de programme informatique comprenant des instructions exécutables par ordinateur permettant d'amener un dispositif de commande (800) à effectuer au moins les opérations suivantes :
- l'enregistrement (101) d'un signal d'électrocardiogramme (201), en abrégé signal ECG ;
- l'enregistrement (102) d'un signal de photopléthysmographie (202), en abrégé signal PPG, de manière semi-synchrone avec ledit enregistrement (101) dudit signal ECG ;
- l'annotion (103) de segments dans ledit signal ECG (201) soit sur la base d'un algorithme, soit sur la base d'un expert, qui conduit à des annotations de segment d'ECG ;
- l'alignement temporel (104) dudit signal PPG (202) et dudit signal ECG (201) ;
- la détection (105) de battements cardiaques (301) dans ledit signal ECG, appelés battements ECG ;
- la détection (106) de battements cardiaques (302) dans ledit signal PPG, appelés battements PPG ;
- l'appariement (107) desdits battements ECG (301) auxdits battements PPG (302) ;
- la dérivation (108) d'annotations pour des segments de signal PPG en fonction de la nature desdites annotations de segment d'ECG et de la façon dont lesdits battements ECG (301) peuvent être appariés auxdits battements PPG (302) ; et
- l'annotation (109) desdits segments de signal PPG à l'aide desdites annotations, ce qui génère ledit signal PPG annoté (402).

15. Support de stockage lisible par ordinateur comprenant des instructions exécutables par ordinateur d'un programme permettant d'effectuer les étapes suivantes lorsque le programme est exécuté sur un ordinateur (800) :
- l'enregistrement (101) d'un signal d'électrocardiogramme (201), en abrégé signal ECG ;
- l'enregistrement (102) d'un signal de photopléthysmographie (202), en abrégé signal PPG, de manière semi-synchrone avec ledit enregistrement (101) dudit signal ECG ;
- l'annotion (103) de segments dans ledit signal ECG (201) soit sur la base d'un algorithme, soit sur la base d'un expert, qui conduit à des annotations de segment d'ECG ;
- l'alignement temporel (104) dudit signal PPG (202) et dudit signal ECG (201) ;
- la détection (105) de battements cardiaques (301) dans ledit signal ECG, appelés battements ECG ;
- la détection (106) de battements cardiaques (302) dans ledit signal PPG, appelés battements PPG ;
- l'appariement (107) desdits battements ECG (301) auxdits battements PPG (302) ;
- la dérivation (108) d'annotations pour des segments de signal PPG en fonction de la nature desdites annotations de segment d'ECG et de la façon dont lesdits battements ECG (301) peuvent être appariés auxdits battements PPG (302) ; et
- l'annotation (109) desdits segments de signal PPG à l'aide desdites annotations, ce qui génère ledit signal PPG annoté (402).
